# EUROPEAN PATENT SPECIFICATION

(11) **EP 3 258 944 B1**
(45) Date of publication and mention of the grant of the patent: **14.10.2020**
(21) Application number: 15834768.2
(22) Date of filing: 20.11.2015
(51) Int. Cl.: A61K 35/50, C12N 5/071, A61P 35/00, A61P 27/02

(54) **POPULATION OF AMNIOCYTES HAVING PHAGOCYTIC ACTIVITY AGAINST PATHOLOGICAL CELLS, PROCESS FOR THE PREPARATION THEREOF AND USES THEREOF IN THE MEDICAL FIELD**
POPULATION VON AMNIOZYTEN MIT PHAGOZYTISCHER AKTIVITÄT GEGEN PATHOLOGISCHE ZELLEN, VERFAHREN ZUR HERSTELLUNG DAVON UND VERWENDUNGEN DAVON IN DER MEDIZIN
POPULATION D'AMNIOCYTES PRÉSENTANT UNE ACTIVITÉ PHAGOCYTAIRE CONTRE DES CELLULES PATHOLOGIQUES, PROCÉDÉ POUR LES PRÉPARER ET LEURS UTILISATIONS DANS LE DOMAINE MÉDICAL

(43) Date of publication of application: 27.12.2017
(73) Proprietor: Università degli Studi di Sassari, 07100 Sassari (SS) (IT)
(72) Inventor: FENU PINTORI, Grazia, 07100 Sassari (IT)
(74) Representative: Gitto, Serena
(86) International application number: PCT/IT2015/000281
(87) International publication number: WO 2017/085749

(56) References cited:
- WO-A1-2012/083023
- MARTA MAGATTI ET AL: "Amniotic membrane-derived cells inhibit proliferation of cancer cell lines by inducing cell cycle arrest", JOURNAL OF CELLULAR AND MOLECULAR MEDICINE, vol. 16, no. 9, 23 August 2012 (2012-08-23), pages 2208-2218, XP055052014, ISSN: 1582-1838, DOI: 10.1111/j.1582-4934.2012.01531.x
- KANG N -H ET AL: "Potential antitumor therapeutic strategies of human amniotic membrane and amniotic fluid-derived stem cells", CANCER GENE THERAPY 2012 NATURE PUBLISHING GROUP USA, vol. 19, no. 8, August 2012 (2012-08), pages 517-522, XP002757408, ISSN: 0929-1903
- CHIAVEGATO ET AL: "Human amniotic fluid-derived stem cells are rejected after transplantation in the myocardium of normal, ischemic, immuno-suppressed or immuno-deficient rat", JOURNAL OF MOLECULAR AND CELLULAR CARDIOLOGY, ACADEMIC PRESS, GB, vol. 42, no. 4, 5 April 2007 (2007-04-05), pages 746-759, XP022020007, ISSN: 0022-2828, DOI: 10.1016/J.YJMCC.2006.12.008

## Description

### field

The present invention relates to a population of amniocytes having phagocytic activity against pathological cells, the process for the preparation thereof and uses thereof in the medical field.

In more detail, the invention relates to a population of amniocytes having phagocytic activity against pathological cells, such as tumour cells or the cells of other cellular diseases, such as, for example, proliferative vitreoretinopathy, the process for the preparation thereof and uses thereof in the medical field.

As is well known, there are numerous pathologies that manifest themselves through alterations of the cells of the tissues involved in the pathology itself. Among cellular pathologies, mention can be made, for example, of proliferative vitreoretinopathy and tumours.

Proliferative vitreoretinopathy is an ocular pathology included in the national register of social diseases. It is often the consequence of pre-existing pathology-inducing events such as traumas, eye surgery and diabetes, but a hereditary form also exists. The high prevalence of this disease observed in Sardinia depends on the widespread presence of diabetes in this region, which, as it is well known, ranks second in Europe in terms of incidence. A variable percentage of patients affected by vitreoretinopathy progress inexorably towards complete blindness, often at a young age, regardless of modern therapies.

The vitreous is an organ that can be observed with different investigative techniques. At present, use is made of OCT (optical coherence tomography), an extremely sophisticated, high-resolution radiological examination. However, since the vitreous is a closed body housing only a few cells suspended in liquid, it cannot be easily defined in detail, much less reached with the common administration of drugs. Though some pharmacological solutions are injected into the body, unfortunately the degeneration of this important organ often leads to blindness in both hereditary and acquired vitreoretinopathy.

No safe, effective therapies for vitreoretinopathy exist at present. Among the new therapies being developed, an important role is played by the intraocular injection of triamcinolone. However, the administration of this active ingredient using an invasive technique can result in undesirable effects and possible complications such as: septic endophthalmitis 0.9% (1) or aseptic endophthalmitis (19-21); ocular hypertension 25% (12), 52% (22); subconjunctival and/or vitreous haemorrhage; onset/progression of cataract (up to 60%); retinal detachment, in a percentage that has not yet been defined but can be estimated as 1-5%; pain, eye reddening, foreign body sensation; transitory appearance of moving bodies (free drug crystals in the vitreous chamber).

Promising multicentre, randomised, double-blind clinical studies on therapy with the product MACUGEN® (pegaptanib sodium) are also underway. However, the long-term effects and patient compliance are yet to be ascertained, as this is a therapy to be administered every 6 weeks.

Ranibizumab (RhuFab V2; Lucentis, Genentech, Novartis) is an antibody fragment derived from bevacizumab which binds and blocks all forms of the vascular endothelial growth factor, also referred to as VEGF (VEGF165, VEGF121, VEGF110), in extracellular space. The therapy seeks to eliminate or drastically reduce the growth of vessels in such a way as to prevent opacification of the vitreous and destruction of the retina.

Avastin® (bevacizumab) is a new drug that is used in cancer therapy to block the growth of an anarchic neovascular network within metastatic colorectal tumours. Being an off-label therapy, it can no longer be used against neovascularisation, though it maintains a role in macular oedema. Moreover, current legislation provides for a very limited use of this drug and only within the framework of clinical protocols.

As mentioned above, tumours are likewise pathologies characterised by a cellular alteration. Among tumours, mention can be made, for example, of breast cancer, neuroblastoma, leukaemia, myeloma, prostate cancer and tumours of endocrine origin.

Neuroblastoma, for example, is a malign childhood tumour of neural crest cells, which give rise to the sympathetic nervous system. It represents about 10% of the solid tumours in infants and in children of 15 years of age or less, with a yearly incidence of about 1/70,000 in children belonging to this age group. In 90% of cases, neuroblastoma is diagnosed before 5 years of age. Neuroblastoma is responsible for 15% of deaths attributed to neoplasia in children and in 50% of cases it is already metastatic at the time of diagnosis and unresponsive to chemotherapy. The clinical manifestations of neuroblastoma are highly variable and depend on the stage and site of the tumour, which can develop in any part of the sympathetic nervous system (around 80% of cases develop in the abdomen). Localised forms are discovered by chance or are revealed by the presence of an abdominal or chest mass, which can be associated with pain. At the time of diagnosis, metastatic forms represent about 50% of the cases. The symptoms of metastasis, such as bone pain, claudication, paralysis, hepatomegaly (Pepper syndrome) and exophthalmos (Hutchinson syndrome), are indicative of metastasized neuroblastoma. The disease is also associated with arterial hypertension, fever, an alteration in the general state of health (weight loss, pain, irritability and anaemia) and various genetic defects, which influence the prognosis. Amplification of the MYCN oncogene (2p24.3) is an unfavourable prognostic marker; triploidy and numerical anomalies of chromosomes are associated with a good prognosis, whereas diploidy or tetraploidy and segmental anomalies of chromosomes (including losses of 1p, 11q or duplications of 17q) are associated with an unfavourable prognosis. A mutation of the ALK gene in about 12% of cases has been recently described. The diagnosis is based on identification of an increase in the levels of urinary catecholamine metabolites (VMA, HVA and dopamine) and scanning of the initial tumour by ultrasound imaging, cerebral exploration or magnetic resonance imaging. MIBG (iodine-131-meta-iodobenzylguanidine) scintigraphy and an examination of bone marrow are useful for identifying metastasis. A biopsy of the tumour confirms the diagnosis, enables its histological classification and contributes to identifying MYCN amplification.

Localised forms of neuroblastoma are treated with surgical resection, sometimes preceded by chemotherapy. The treatment of metastatic forms in children less than one year of age and forms exhibiting MYCN amplification is possible with conventional chemotherapy, initial surgical removal of the tumour, high-dose chemotherapy associated with transplantation of haematopoietic stem cells, local radiotherapy and maintenance therapy with retinoic acid. Most localised tumours have an excellent prognosis after surgery. Children less than one year of age have a better prognosis than older children. Some tumours can even regress spontaneously. By contrast, about 60% of children over one year old who are affected by a metastasised tumour have an unfavourable prognosis even when intensive treatments are undertaken. In children over one year old, the survival rate at 5 years varies from 95% for some localised tumours to 30% for metastatic neuroblastoma.

In light of the above, there is an evident need to be able to have new therapies available which can overcome the disadvantages of the known therapies against cellular pathologies.

The solution according to the present invention fits into this context, as it aims to provide a new selective therapy against pathological cells.

Amniocytes are cells which, together with other cellular elements, make up amniotic fluid, i.e. the liquid contained in the amniotic sac, whose main function is to protect an embryo from traumas of a mechanical nature. Today, amniotic fluid also plays an important role from a diagnostic viewpoint. By analysing it, in fact, it is possible to evaluate the development and maturity of a foetus and its state of wellbeing or intrauterine distress through tests like amniocentesis and amnioscopy.

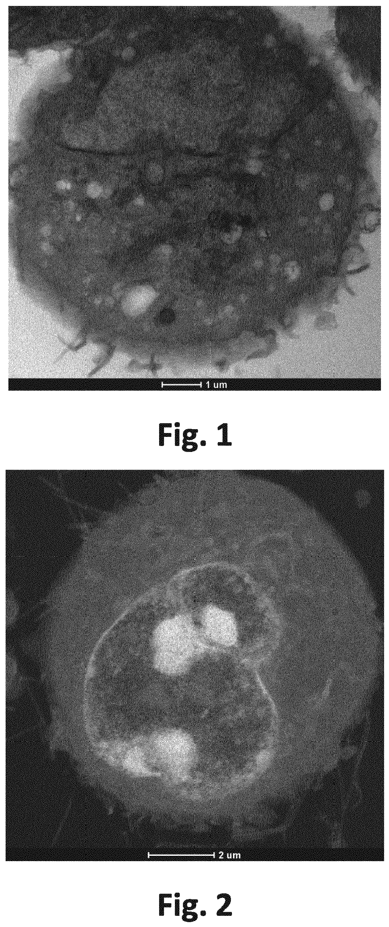

The amniocytes present in amniotic fluid are cells serving to coat the inner wall of the sac, and as such are intended to provide a wall to protect the embryo, and later the foetus. These cells are also visible in amniotic fluid, though not in a detailed manner, when they are cultured for genetic analysis, mixed with sloughed off cells of the embryo. Amniocytes grow extremely slowly when cultured together with other cells and can be distinguished by a peculiar morphology which makes them very different from "classic stem cells" with a spindle-like appearance. In fact, amniocytes are rounded and the surface of the cell membrane is extremely jagged. Amniocytes are thus characterised by a globose shape, with a cytoplasmic membrane exhibiting protuberances that vary in number and shape based on the stage of maturity. The nucleus, rather voluminous, sometimes appears rounded, but in most cases is ovoid with a very evident nuclear membrane having an irregular structure and it is provided with a nucleolus. The best observations have been made with a transmission electron microscope, as may be seen in the photos shown in figures 1 and 2.

According to the present invention, a method has now been developed which enables amniocytes to be made to acquire a selective phagocytic activity against pathological cells. Therefore, the population of amniotic cells having phagocytic activity according to the present invention can be advantageously employed in the treatment of cellular pathologies. In particular, it has been observed that amniotic cells (or amniocytes) are CD68+ like macrophages. Moreover, amniotic cells cultured together with pathological cells, such as, for example, pathological hyalocytes from the vitreous excised from patients with proliferative vitreoretinopathy, acquire, and maintain over time, an ability to phagocytise the pathological cells they were co-cultured with previously. The same phenomenon of acquisition of phagocytic activity by amniotic cells has been observed in co-culture with tumour cells of different types of tumours, such as, for example, breast cancer and neuroblastoma.

Following activation of the phagocytic ability, if the "activated" amniocytes are placed in contact with cells of the same pathology, they act immediately by phagocytising the pathological cells in half the time compared to the first co-culture times. Therefore, the amniocytes in which a phagocytic ability has been activated acquire a memory of the pathology whose cells they were co-cultured with. It is thus possible to prepare a database in which discarded amniotic fluids, previously used for diagnosis by amniocentesis, are activated with the cells of a whole variety of pathologies and cryopreserved until they are used in therapy.

The examples given below describe only a few studies conducted with different types of pathological cells. It can be affirmed with absolute certainty that the phagocytic ability of amniocytes is effective against the cellular proliferation of tumours such as leukaemia, myeloma, malignant prostate cancer and tumours of endocrine origin.

Therefore, the subject matter of the present invention specifically relates to a population of phagocytic amniocytes of amniotic fluid having selective phagocytic activity against pathological cells of a cellular pathology, wherein said population of phagocytic amniocytes of amniotic fluid is obtained by co-culturing amniocytes of amniotic fluid with pathological cells of a cellular pathology for a period of time equal to at least the time that elapses until the start of the phagocytic activity on the part of the amniocytes against said pathological cells, wherein the amniocytes co-cultured with pathological cells have a morphology characterised by a rounded shape and jagged cell membrane when observed by transmission electron microscope and do not have phagocytic activity and wherein the cellular pathology is proliferative vitreoretinopathy or tumours.

The population of phagocytic amniocytes of amniotic fluid according to the present invention can be advantageously used in the medical field.

Therefore, the present invention relates to a pharmaceutical composition comprising a population of phagocytic amniocytes of amniotic fluid according to the present invention, as an active ingredient, together with one or more excipients and/or adjuvants.

Moreover, the present invention relates to the population of phagocytic amniocytes of amniotic fluid or the pharmaceutical composition, as defined above, for use in the medical field.

More particularly, the population of phagocytic amniocytes of amniotic fluid according to the present invention or the pharmaceutical composition as defined above can be employed in the treatment of pathologies such as proliferative vitreoretinopathy and tumours.

The subject matter of the present invention further relates to a process for preparing the population of phagocytic amniocytes of amniotic fluid as defined above, said process comprising or consisting in a step of co-culturing amniocytes of amniotic fluid with pathological cells of a cellular pathology for a period of time equal to at least the time that elapses until the start of phagocytic activity on the part of the amniocytes of amniotic fluid against said pathological cells, wherein the amniocytes co-cultured with pathological cells have a morphology characterised by a rounded shape and jagged cell membrane when observed by transmission electron microscope and do not have phagocytic activity and wherein the cellular pathology is proliferative vitreoretinopathy or tumours.

According to a particular embodiment of the present invention, the amniocytes of amniotic fluid are co-cultured with the pathological cells in a ratio of at least 100 amniotic cells to every 10 pathological cells, such as, for example hyalocytes from a vitreous with proliferative vitreoretinopathy or tumour cells.

The present invention will now be described, by way of non-limiting illustration, with particular reference to several illustrative examples and the figures in the appended drawings, in which:
Figure 1 shows an amniocyte observed under a transmission electron microscope; one may note the jagged cytoplasmic membrane, rich in protuberances; inside the cell, the cellular organelles and the evident nucleus.
Figure 2 shows an amniocyte observed under a transmission electron microscope; the nucleus with the characteristic "kidney" shape and chromatin arranged in clumps (light areas) are evident; the nuclear membrane is also clearly evident.
Figure 3 shows hyalocytes of the vitreous body obtained from an excised eyeball. The elongated cell at the centre is a Muller cell, an occasionally found element originating from the retina; granules of melanin can be observed.
Figure 4 shows the presence of amniocytes and hyalocytes in culture after 3 hours.
Figure 5 shows the production of hyaluronic gel by healthy hyalocytes and the amniocytes that surround some altered hyalocytes after 24 hours.
Figure 6 shows the production of a hyaluronic matrix and the presence of amniocytes which engulf some pathological hyalocytes after 36 hours.
Figure 7 shows that the amniocytes in co-culture with hyalocytes express CD68 like in the controls; plate obtained after 12 hours of culture, as an immunohistochemical control.
Figure 8 shows cells with small and prominent nuclei (hyalocytes) and rounded, elongated cells with an irregular surface (amniocytes); plate obtained after 24 hours of culture; haematoxylin and eosin staining.
Figure 9 shows that the altered hyalocytes are brought together by the amniocytes prior to phagocytosis; plate obtained after 36 hours of culture; haematoxylin and eosin staining.
Figure 10 shows amniocytes with a cytoplasm that is extremely rich in vacuoles after phagocytosis, the production of hyaluronic gel and vitreal matrix, as in a normal vitreous; plate obtained after 72 hours of culture; haematoxylin and eosin staining.
Figure 11 shows hyalocytes immersed in hyaluronic matrix and melanin pigments. Disappearance of the altered hyalocytes. Plate obtained after 96 hours of culture. Haematoxylin and eosin staining.
Figure 12 shows the co-culture in which the immunohistochemical staining reveals the CD68+ amniocytes and altered vitreal hyalocytes.
Figure 13 shows the co-culture in which the immunohistochemical staining reveals the CD68+ amniocytes and the normal vitreal hyalocytes are barely visible.
Figure 14 shows MCF-7 cells in culture; the cells have reached confluence and are ready to be co-cultured.
Figure 15 shows the co-culture after 24 hours, with amniocytes that are elongated or rounded, but have evident extensions of the cytoplasmic membrane, stretching toward the MCF-7 cells, characterised by a particularly prominent round nucleus in which the nucleolus can be easily seen.
Figure 16 shows round cells of a bright yellow colour, with nucleus and nucleolus (MCF-7), and elongated amniocytes stretching towards the tumour cells after 12 hours.
Figure 17 shows that, after 24 hours of co-culture, the amniocytes enter into contact with the tumour cells.
Figure 18 shows that, after 36 hours, the cytoplasm of the amniocytes appears rich in numerous granules of a bright white colour, like the one characterising the tumour cells.
Figure 19 shows an amniocyte with its cytoplasm completely filled with bright white material, after 48 hours of co-culture.
Figure 20 shows the MCF-7 cells that are inside the amniocyte, or confluent amniocytes, after 72 hours of co-culture.
Figure 21 shows that, after 96 hours of co-culture, the amniocytes show a cytoplasm rich in small, bright white granules and the rounded shape that characterises them when they are in culture prior to phagocytosis. Confluent amniocytes can be noted on the right.
Figure 22 shows, after 96 hours, rounded amniocytes and one in the process of changing morphology from elongated to rounded. In both images the cytoplasm contains bright white granules.
Figure 23 shows SH-SY5Y tumour cells in culture, at confluence. Note the characteristic spindle-like appearance, the clearly evident nucleus and long neurites.
Figure 24 shows a co-culture of amniocytes that are both round and spindle-shaped in appearance after 36 hours. The small spindle-shaped tumour cells appear to be surrounded by amniocytes.
Figure 25: after 72 hours, no tumour cells are visible. Some amniocytes form a syncytium. A few scattered nuclei belonging to the tumour cells remain in the medium.

### EXAMPLE 1: Method for preparing a population of amniotic cells having phagocytic activity according to the present invention against pathological cells

The amniocytes are obtained from discarded amniotic fluid culture specimens from prenatal diagnosis, said amniotic fluid being collected through amniocentesis and cultured for three weeks for diagnostic purposes.

The amniocytes are cultured for 3 days in Dulbecco's Modified Eagle Medium (DMEM) in order to enable the cells to better adapt and above all to restore their physiological parameters. The 3-day culture sometimes requires a 1- or 2-day extension with a change of culture medium at 24 hours each day. This is because in the discarded cultures from the prenatal diagnosis the amniocytes are in a state of metabolic impoverishment, due to the fact that the culture medium has not been changed, keeping alive cells from which diagnostic responses have already been obtained being no longer necessary.

Before being revitalised, the amniocytes contained in the culture flasks are subjected to trypsinization, that is, separated from one another and placed in a water and glycerol gradient and centrifuged at 500 rpm for about 30 minutes to eliminate all the fibroblasts that could prejudice the outcome of the co-culture. In fact, fibroblasts reproduce at an extremely fast rate and reach confluence, i.e. come to occupy the entire culture plate, in just a few days, especially if nourished with a daily change of culture medium.

The band of cells corresponding to the amniocytes is highlighted with a blue lamp. The cells are immediately placed into culture in a specific medium (TopGrow, by the company Resnova) and left to adapt until they reach a sufficient number, generally about 200,000, or until the cells reach confluence. The number of amniocytes can vary based on the quantity of cells originally present in the discarded cultures and the recovery capacity of the amniocytes. However, a sufficiently large number of cells is generally reached, such as to enable the collection of about 100 amniocytes to be co-cultured with about 10 pathological cells. Amniocytes are few in number in the cultures obtained from amniocentesis and their ability to divide is extremely slow, so they require constant and abundant nourishment.

The amniocyte culture can be preserved by freezing, for example at -80°C, or else it can be used directly to prepare amniocytes with phagocytic activity by co-culturing the amniocytes with pathological cells.

During co-culture, daily observation is fundamental in order to observe when the amniocytes begin phagocytising the pathological cells.

### EXAMPLE 2: In vitro study on the phagocytic activity of the amniocytes according to the present invention against pathological cells of proliferative vitreoretinopathy

About 100 amniocytes obtained according to example 1 were cultured with about 10 hyalocytes from a pathological vitreous excised due to vitreoretinopathy.

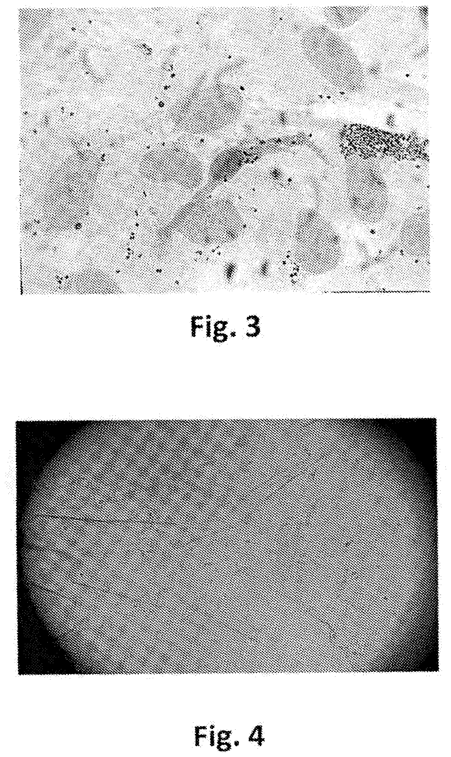

The vitreous was subjected to centrifugation (3,000 rpm for 30 - 40 minutes) in order to recover the hyalocytes, which, being in an extremely small number, are difficult to be isolated. Once concentrated on the bottom of the test tube, the hyalocytes are immediately introduced into the same culture well (TopGrow culture medium by Resnova) in which the amniocytes have already been placed and the culture is left at a controlled temperature of 36° until the time of observation, about 24 hours later. The number of cultures carried out per case is never less than 3 wells, because it has to be foreseen that some cells, amniocytes and/or hyalocytes, for reasons that cannot always be explained, may not find ideal conditions for growth and it is thus preferable to have a sufficiently large number of cultures (fig.4).

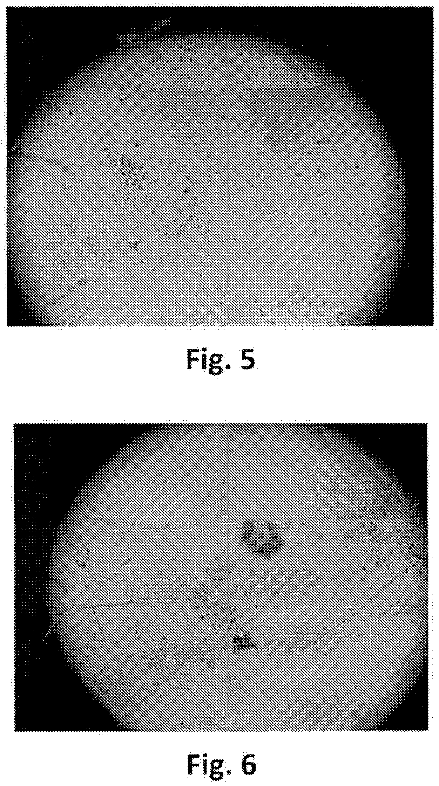

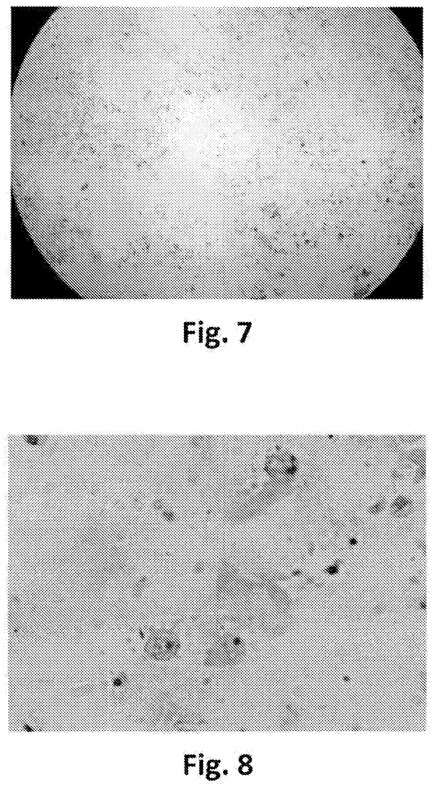

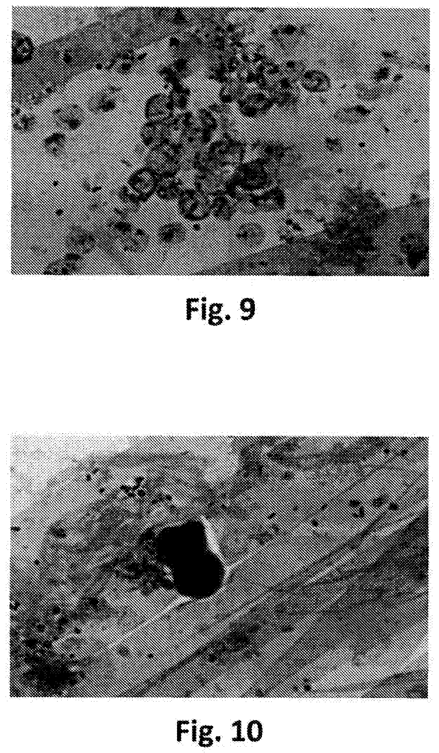

The results obtained from the co-cultures of amniocytes and hyalocytes brought to light some interesting aspects, which confirm not only the macrophage-like activity of the amniocytes, as proven by their positivity to CD68, but also that during vitreoretinopathy the vitreous contains altered hyalocytes within it (see comparison between figs. 3 and 8). The pathological hyalocytes are characterised by having a thickened cell membrane and a rounded appearance differing from the characteristic cell morphology with a light-coloured cytoplasm containing few organelles. After just 24 hours of co-culture, one witnesses a phenomenon of the amniocytes "encircling" the vitreal cells (fig.5). Encircling is undoubtedly followed by a phenomenon of phagocytosis of vitreal cells by the amniocytes, which tend to become elongated almost as if to prepare to "embrace" the cells to be phagocytised. In a total time of about 72 hours, and even more so after 96 hours, in the culture it is possible to observe a resumption of the activity of hyaluronic acid matrix synthesis by healthy hyalocytes that had been present in the culture but were overwhelmed by the pathological ones (fig. 6-11). The vitreous body is a closed structure that acts like a lens, and its opacification creates blindness. Irrespective of what triggered the disease, pathological hyalocytes also cease producing hyaluronic acid, a fundamental medium in which these cells are immersed and from which they derive, or at least are assumed to derive, nourishment. The production of this matrix in vitro is a sign of resumption of the normal activity of healthy hyalocytes, which, in the event of pathology, are overwhelmed by pathological cells. The phagocytic activity of the amniocytes is directed not only at pathological vitreal cells, but also at the melanin granules that may be found in the midst of the hyalocytes when they are isolated.

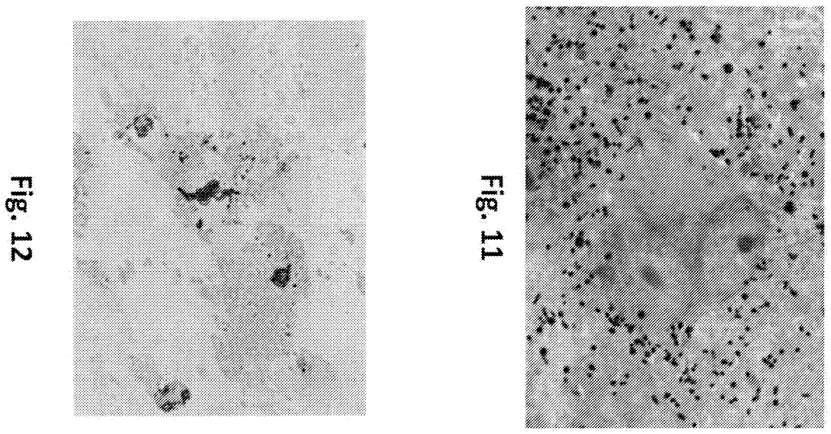

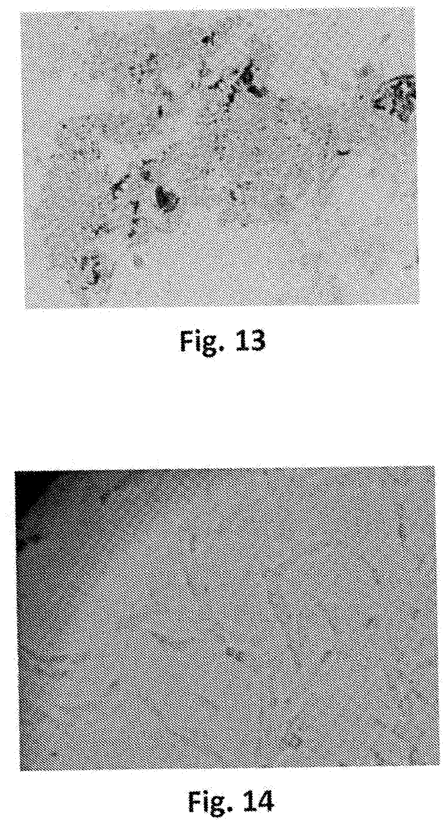

A further immunohistochemical test was carried out to verify whether the amniocytes maintained their "macrophagic status" at a more advanced stage of co-culture, figs.12 and 13.

Based on the experiments performed on about 90 vitreous bodies excised due to vitreoretinopathy and the results obtained, it appears evident that the amniocytes are capable of eliminating pathological hyalocytes within the vitreous body, cleaning the body itself of cells that, due to various causes, destroy the transparency of this important lens of the organ of vision.

### EXAMPLE 3: In vitro study on the phagocytic activity of the amniocytes according to the present invention against pathological cells of breast cancer

Approximately 100 amniocytes obtained as per example 1 were cultured (TopGrow culture medium) with about 10 malignant human breast tumour cells of the MCF-7 cell line (Altogen Labs).

MCF-7 cells are cells that are kept frozen at -80°C and not thawed until the time of experimentation. At least 3-4 days of adaptation in an incubator are necessary and the cells must reach confluence, i.e. they must occupy the whole surface of the culture plate (diameter of about 3 cm) before they can be collected (fig.14).

On reaching confluence, the MCF-7 cells are counted and 10 are introduced onto the culture plate, where they will come into contact with the amniocytes, present on the plate in a number of 100. The number of cultures per experiment is never less than 3 wells, because it has to be foreseen that some cells, amniocytes and/or MCF-7, but almost always amniocytes, for reasons that cannot always be explained, may not find ideal conditions for growth and it is thus preferable to have a sufficiently large number of cultures.

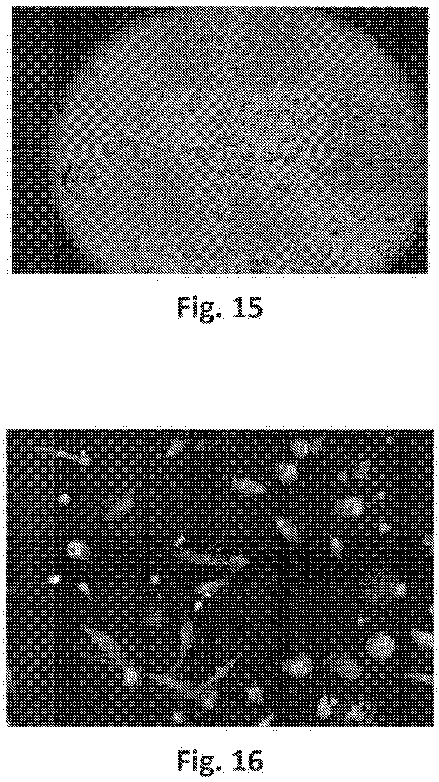

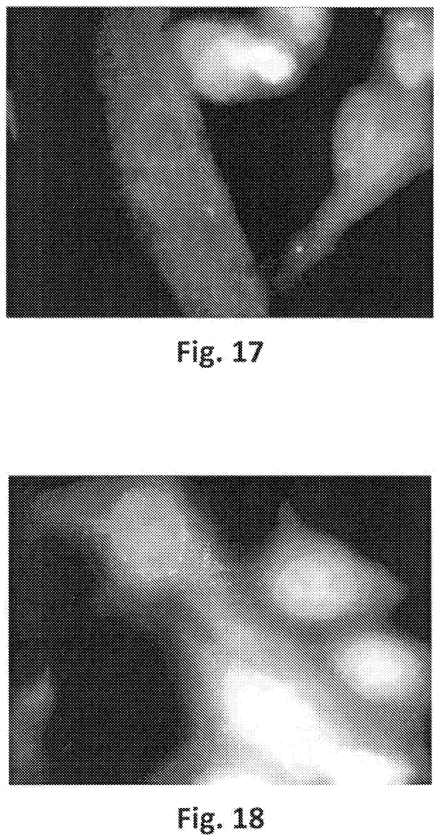

From the results obtained, it appears clear that the amniocytes, on contact with the MCF-7 cells, are capable of picking up a signal of a "foreign" cell and activating a series of morphological changes. The cells become elongated, almost as if to suggest a sort of diapedesis, or cellular movement, which is already known in macrophages, in order to reach the "enemy" cells (fig.15). The change in the configuration of the amniocyte shows the ability of these cells to surround tumour cells (fig.16) and then engulf them. Subsequently, no whole tumour cell is any longer visible, only bright yellow tumour cell fragments inside the cytoplasm of the amniocytes (figs. 17-22).

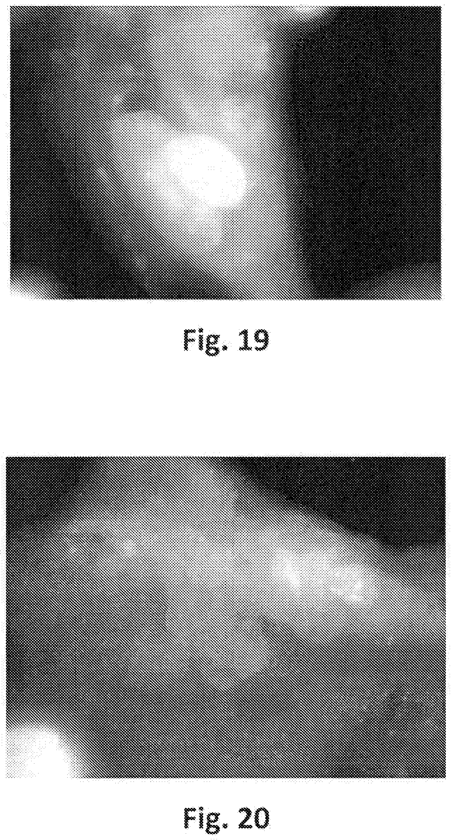

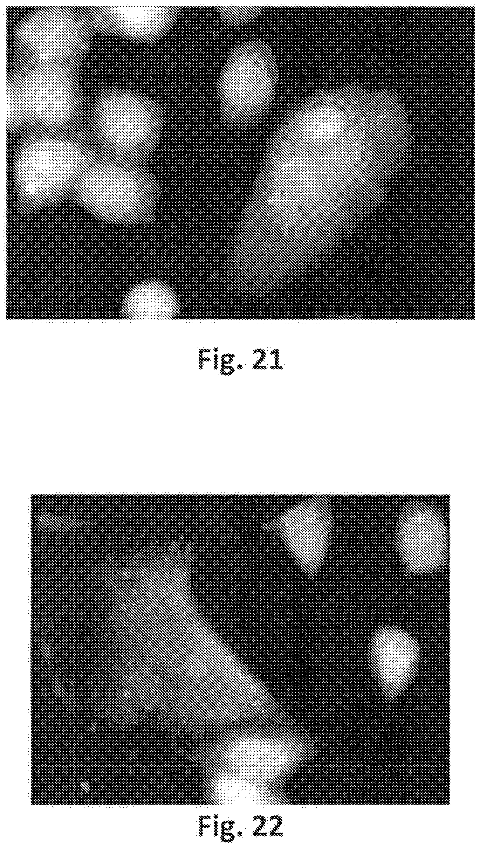

It is interesting to note that the amniocytes join together in a syncytium, that is, they unite their cytoplasm while maintaining every nucleus intact (figs. 20 and 21). This phenomenon was not observed in the co-cultures with hyalocytes. The phenomenon is part of the process of phagocytosis carried out by macrophages. The amniocytes that join together in a syncytium can be considered activated and hence capable of undertaking this same action every time they are together with MCF-7 cells, cells of the malignant human breast tumour cell line, which are estrogen receptor-negative.

### EXAMPLE 4: In vitro study on the phagocytic activity of the amniocytes according to the present invention against pathological cells of neuroblastoma

Approximately 100 amniocytes obtained as per example 1 were cultured (TopGrow culture medium) with about 10 cells of the SH-SY5Y neuroblastoma cell line (SH-SY5Y human cell line, SIGMA-ALDRICH).

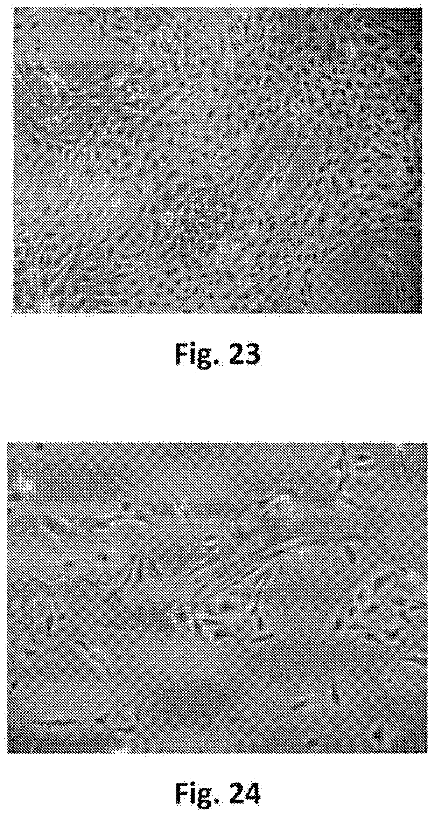

The original cell line, called SK-NSH, is the line from which the subclone SH-SY5Y derives. These cells propagate by mitosis and show long neurites, which stretch out in the areas surrounding the cells. During the process of division they tend to form "clusters", which clearly reveal their origin as tumour cells (fig.23).

SH-SY5Y cells are cells that are kept frozen at -80°C and not thawed until the time of experimentation. At least 3-4 days of adaptation in an incubator are necessary and the cells must reach confluence, i.e. they must occupy the whole surface of the culture plate (diameter of about 3 cm) before they can be collected.

On reaching confluence, the SH-SY5Y cells are counted and 10 are introduced onto the culture plate, where they will come into contact with the amniocytes, present on the plate in a number of 100. The number of cultures per experiment is never less than 3 wells, because it has to be foreseen that some cells, amniocytes and/or SH-SY5Y, but almost always amniocytes, for reasons that cannot always be explained, may not find ideal conditions for growth and it is thus preferable to have a sufficiently large number of cultures.

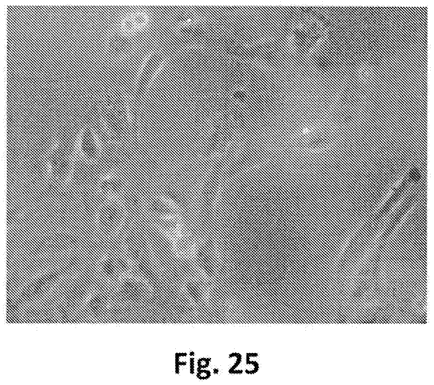

SH-SY5Y tumour cells, extremely malignant, appear to succumb to amniocytes, so much so that after 72 hours of co-culture it is no longer possible to see any presence. It was not possible to document the events in sequence, since, despite their high capacity for multiplication, the SH-SY5Y cells are phagocytised by the amniocytes in just a few hours (fig.24). Fig.25 shows the amniocytes in a syncytium or isolated after 72 hours of co-culture; their cytoplasm appears swollen with granules, an appearance that is more than evident if compared with the cytoplasm of the amniocytes in fig.24, which is lighter and devoid of granulation.

## Claims

1. A population of phagocytic amniocytes of amniotic fluid having selective phagocytic activity against pathological cells of a cellular pathology, wherein said population of phagocytic amniocytes of amniotic fluid is obtained by co-culturing amniocytes of amniotic fluid with pathological cells of a cellular pathology for a period of time equal to at least the time that elapses until the start of the phagocytic activity on the part of the amniocytes against said pathological cells, wherein the amniocytes co-cultured with pathological cells have a morphology **characterised by** a rounded shape and jagged cell membrane when observed by transmission electron microscope and do not have phagocytic activity and wherein the cellular pathology is proliferative vitreoretinopathy or tumours.

2. A population of phagocytic amniocytes of amniotic fluid according to claim 1, wherein the amniocytes co-cultured with pathological cells have a morphology as shown in figures 1 and 2.

3. A pharmaceutical composition comprising or consisting of the population of phagocytic amniocytes of amniotic fluid according to anyone of claims 1-2, as an active ingredient, together with one or more excipients and/or adjuvants.

4. The population of phagocytic amniocytes of amniotic fluid according to anyone of claims 1-2 or pharmaceutical composition according to claim 3, for use as a medicament.

5. The population of phagocytic amniocytes of amniotic fluid according to anyone of claims 1-2 or the pharmaceutical composition according to claim 3, for use in the treatment of proliferative vitreoretinopathy.

6. The population of phagocytic amniocytes of amniotic fluid according to anyone of claims 1-2 or the pharmaceutical composition according to claim 3, for use in the treatment of tumours.

7. A process for preparing the population of phagocytic amniocytes of amniotic fluid according to anyone of claims 1-2, said process comprising or consisting in a step of co-culturing amniocytes of amniotic fluid with pathological cells of a cellular pathology for a period of time equal to at least the time that elapses until the start of the phagocytic activity on the part of the amniocytes of amniotic fluid against said pathological cells, wherein the amniocytes co-cultured with pathological cells have a morphology **characterised by** a rounded shape and jagged cell membrane when observed by transmission electron microscope and do not have phagocytic activity and wherein the cellular pathology is proliferative vitreoretinopathy or tumours.

8. The process according to claim 7, wherein the amniocytes co-cultured with pathological cells have a morphology as shown in figures 1 and 2.

9. The process according to anyone of claims 7-8, wherein the amniocytes of amniotic fluid are co-cultured with the pathological cells in a ratio of at least 100 amniotic cells to every 10 pathological cells.

10. The process according to anyone of claims 7 and 9, wherein said pathological cells of a cellular pathology are selected from the group consisting of hyalocytes from a vitreous with proliferative vitreoretinopathy or tumour cells.

## Patentansprüche

1. Population von phagozytischen Amniozyten von Amnionflüssigkeit, die selektive phagozytische Aktivität gegen pathologische Zellen einer zellulären Pathologie aufweist, wobei die Population phagozytischer Amniozyten von Amnionflüssigkeit erhalten wird, indem Amniozyten von Amnionflüssigkeit mit pathologischen Zellen einer zellulären Pathologie für eine Zeitdauer gleich mindestens der Zeit, die bis zum Beginn der phagozytischen Aktivität auf dem Teil der Amniozyten gegen die pathologischen Zellen vergeht, co-kultiviert werden, wobei die mit pathologischen Zellen co-kultivierten Amniozyten eine Morphologie aufweisen, die durch eine abgerundete Form und gezackte Zellmembran gekennzeichnet ist, wenn durch Durchstrahlungselektronenmikroskop beobachtet, und sie keine phagozytische Aktivität aufweisen und wobei die zelluläre Pathologie proliferative Vitreoretinopathie oder Tumore ist.

2. Population von phagozytischen Amniozyten von Amnionflüssigkeit nach Anspruch 1, wobei die mit pathologischen Zellen co-kultivierten Amniozyten eine wie in Figur 1 und 2 gezeigte Morphologie aufweisen.

3. Pharmazeutische Zusammensetzung, die die Population von phagozytischen Amniozyten von Amnionflüssigkeit nach einem der Ansprüche 1-2 als einen Wirkstoff gemeinsam mit einem oder mehreren Exzipienten und/oder Adjuvantien umfasst oder daraus besteht.

4. Population von phagozytischen Amniozyten von Amnionflüssigkeit nach einem der Ansprüche 1-2 oder pharmazeutische Zusammensetzung nach Anspruch 3 zur Verwendung als ein Medikament.

5. Population von phagozytischen Amniozyten von Amnionflüssigkeit nach einem der Ansprüche 1-2 oder die pharmazeutische Zusammensetzung nach Anspruch 3 zur Verwendung bei der Behandlung von proliferativer Vitreoretinopathie.

6. Population von phagozytischen Amniozyten von Amnionflüssigkeit nach einem der Ansprüche 1-2 oder die pharmazeutische Zusammensetzung nach Anspruch 3 zur Verwendung bei der Behandlung von Tumoren.

7. Prozess zur Vorbereitung der Population von phagozytischen Amniozyten von Amnionflüssigkeit nach einem der Ansprüche 1-2, wobei der Prozess einen Schritt zum Co-kultivieren von Amniozyten von Amnionflüssigkeit mit pathologischen Zellen einer zellulären Pathologie für eine Zeitdauer gleich mindestens der Zeit, die bis zum Beginn der phagozytischen Aktivität auf dem Teil der Amniozyten von Amnionflüssigkeit gegen die pathologischen Zellen vergeht, umfasst oder aus diesem besteht, wobei die mit pathologischen Zellen co-kultivierten Amniozyten eine Morphologie aufweisen, die durch eine abgerundete Form und gezackte Zellmembran gekennzeichnet ist, wenn durch Durchstrahlungselektronenmikroskop beobachtet, und sie keine phagozytische Aktivität aufweisen und wobei die zelluläre Pathologie proliferative Vitreoretinopathie oder Tumore ist.

8. Prozess nach Anspruch 7, wobei die mit pathologischen Zellen co-kultivierten Amniozyten eine wie in Figur 1 und 2 gezeigte Morphologie aufweisen.

9. Prozess nach einem der Ansprüche 7-8, wobei die Amniozyten von Amnionflüssigkeit mit den pathologischen Zellen in einem Verhältnis von mindestens 100 amniotischen Zellen zu 10 pathologischen Zellen co-kultiviert sind.

10. Prozess nach einem der Ansprüche 7 und 9, wobei die pathologischen Zellen einer zellulären Pathologie aus der Gruppe ausgewählt sind, bestehend aus Hyalozyten von einem Glaskörper mit proliferativer Vitreoretinopathie oder Tumorzellen.

## Revendications

1. Population d'amniocytes phagocytaires du liquide amniotique présentant une activité phagocytaire sélective contre des cellules pathologiques d'une pathologie cellulaire, dans laquelle ladite population d'amniocytes phagocytaires du liquide amniotique est obtenue en mettant en co-culture des amniocytes du liquide amniotique et des cellules pathologiques d'une pathologie cellulaire pendant une durée égale à au moins le temps qui s'écoule jusqu'au début de l'activité phagocytaire des amniocytes contre lesdites cellules pathologiques, dans laquelle les amniocytes mis en co-culture avec des cellules pathologiques présentent une morphologie **caractérisée par** une membrane cellulaire de forme arrondie et irrégulière lorsqu'observée avec un microscope électronique par transmission et ne présentent pas d'activité phagocytaire et dans laquelle la pathologie cellulaire est une vitréorétinopathie proliférante ou des tumeurs.

2. Population d'amniocytes phagocytaires du liquide amniotique selon la revendication 1, dans laquelle les amniocytes mis en co-culture avec des cellules pathologiques présentent une morphologie telle que représentée dans les figures 1 et 2.

3. Composition pharmaceutique comprenant ou consistant en la population d'amniocytes phagocytaires du liquide amniotique selon l'une quelconque des revendications 1 à 2, telle qu'un ingrédient actif, conjointement avec un ou plusieurs excipients et/ou adjuvants.

4. Population d'amniocytes phagocytaires du liquide amniotique selon l'une quelconque des revendications 1 à 2 ou composition pharmaceutique selon la revendication 3, pour une utilisation comme médicament.

5. Population d'amniocytes phagocytaires du liquide amniotique selon l'une quelconque des revendications 1 à 2 ou composition pharmaceutique selon la revendication 3, pour une utilisation dans le traitement d'une vitréorétinopathie proliférante.

6. Population d'amniocytes phagocytaires du liquide amniotique selon l'une quelconque des revendications 1 à 2 ou composition pharmaceutique selon la revendication 3, pour une utilisation dans le traitement de tumeurs.

7. Processus de préparation de la population d'amniocytes phagocytaires du liquide amniotique selon l'une quelconque des revendications 1 à 2, ledit processus comprenant ou consistant en une étape de mise en co-culture d'amniocytes du liquide amniotique et de cellules pathologiques d'une pathologie cellulaire pendant une durée égale à au moins le temps qui s'écoule jusqu'au début de l'activité phagocytaire des amniocytes du liquide amniotique contre lesdites cellules pathologiques, dans lequel les amniocytes mis en co-culture avec des cellules pathologiques présentent une morphologie **caractérisée par** une membrane cellulaire de forme arrondie et irrégulière lorsqu'observée avec un microscope électronique par transmission et ne présentent pas d'activité phagocytaire et dans lequel la pathologie cellulaire est une vitréorétinopathie proliférante ou des tumeurs.

8. Processus selon la revendication 7, dans lequel les amniocytes mis en co-culture avec des cellules pathologiques présentent une morphologie telle que représentée dans les figures 1 et 2.

9. Processus selon l'une quelconque des revendications 7 à 8, dans lequel les amniocytes du liquide amniotique sont mis en co-culture avec des cellules pathologiques dans un rapport d'au moins 100 cellules amniotiques sur toutes les 10 cellules pathologiques.

10. Processus selon l'une quelconque des revendications 7 et 9, dans lequel lesdites cellules pathologiques d'une pathologie cellulaire sont choisies dans le groupe consistant en hyalocytes d'une humeur vitrée présentant une vitréorétinopathie proliférante ou des cellules cancéreuses.
